# EUROPÄISCHE PATENTANMELDUNG

(11) **EP 1 647 236 A1**
(43) Veröffentlichungstag der Anmeldung: **19.04.2006**
(21) Anmeldenummer: 04024599.5
(22) Anmeldetag: 15.10.2004
(51) Int. Cl.: A61B 19/00

(54) **Vorrichtung und Verfahren zur Lageüberprüfung von Markern**

(71) Anmelder: BrainLAB AG, 85551 Kirchheim/Heimstetten (DE)
(72) Erfinder: Kluzik, Dr. Jacek, 81373 München (DE); Immerz, Martin, 82166 Gräfelfing (DE); Bodensteiner, Christoph, 81369 München (DE); Brack, Christian Dr., 86420 Diedorf (DE)
(74) Vertreter: Schwabe - Sandmair - Marx

(57) **Zusammenfassung**

Die vorliegende Erfindung betrifft ein Verfahren zur Erkennung einer Positionsänderung mindestens eines an einem ersten Objekt (7) angebrachten ersten Markers oder Referenzsternes (5) relativ zu dem ersten Objekt (7), wobei die Lage mindestens eines ersten charakteristischen Punktes (10) des ersten Objekts (7) mittels des daran befestigten ersten Markers oder Referenzsterns (5) ermittelt wird, die Lage mindestens eines zweiten charakteristischen Punktes (11) eines mit dem ersten Objekt (7) verbundenen zweiten Objekts (8) ermittelt wird und aus der ermittelten Lage der mindestens zwei charakteristischen Punkte (10, 11) bestimmt wird, ob sich die Position des mindestens einen ersten Markers oder Referenzsterns (5) relativ zu dem ersten Objekt (7), an welchem der erste Marker oder Referenzstern (5) befestigt ist, geändert hat; sowie eine Vorrichtung (1), welche das Verfahren zur Erkennung einer Positionsänderung mindestens eines an einem ersten Objekt (7) angebrachten ersten Markers oder Referenzsterns (5) relativ zu einem ersten Objekt (7) durchführen kann.

## Beschreibung

Die vorliegende Erfindung betrifft ein Verfahren und eine Vorrichtung zur Erkennung einer Positionsänderung mindestens eines Markers oder Referenzsterns, insbesondere im medizinischen Bereich, wobei der Marker oder Referenzstern an einem Objekt, wie einem Oberschenkelknochen (Femur), angebracht sein kann, welches mit einem zweiten Objekt, wie einem Unterschenkelknochen (Tibia), verbunden ist. Aus der Lage charakteristischer Punkte oder Achsen durch charakteristische Punkte der Objekte oder Knochen kann ermittelt werden, ob sich die Position des mindestens einen Markers oder Referenzsterns relativ zu dem Objekt, an welchem der Marker oder Referenzstern befestigt ist, geändert hat.

Die DE 694 32 961 T2 und die DE 695 28 998 T2 beschreiben Anordnungen zur Bestimmung der gegenseitigen Lage von Körpern.

Wird ein Marker oder Referenzstern an einem Objekt, wie einem Knochen, z. B. mittels eines einzigen in den Knochen eingesetzten Objektes oder Drahtes befestigt, wie z. B. in der DE 201 03 416 U1 der Anmelderin beschrieben, so kann der Referenzstern während einer Operation, zum Beispiel durch die Berührung eines Arztes unbeabsichtigt verdreht werden und kann somit nicht mehr eine exakte Positionsbestimmung eines Körperteils oder einer Körperstruktur gewährleisten. Dies kann unter anderem dazu führen, dass ein Navigations- oder Erfassungssystem zur Erfassung der Position des Körperteils oder der Körperstruktur eine falsche oder ungenaue Lage des Körperteils oder der Körperstruktur ermittelt.

Eine Vorschrift der FDA besagt, dass ein Chirurg während einer computergestützten navigierten Operation in etwa alle 30 Minuten durch ein Warnsignal daran erinnert wird, den korrekten Sitz der an den Knochen oder anderen Körperteilen befestigten Marker zu überprüfen. Bei dieser Überprüfung muss somit auf die richtige und nur in relativ großen Zeitabständen abgefragte subjektive Einschätzung des behandelnden Arztes vertraut werden.

Es ist eine Aufgabe der vorliegenden Erfindung, ein Verfahren und eine Vorrichtung vorzuschlagen, mit welchen eine schnelle und zuverlässige Erkennung einer Positionsänderung mindestens eines an einem Objekt angebrachten Markers oder Referenzsterns relativ zu dem Objekt, an welchem der Marker oder Referenzstern angebracht ist, möglich ist.

Diese Aufgabe wird durch die Gegenstände der unabhängigen Ansprüche gelöst. Vorteilhafte Ausführungsformen ergeben sich aus den abhängigen Ansprüchen.

Bei dem erfindungsgemäßen Verfahren ist an einem ersten Objekt, z. B. an einem ersten Knochen, wie zum Beispiel dem Schienbeinknochen bzw. der Tibia oder dem Oberschenkelknochen bzw. Femur eines Patienten, zum Beispiel vor oder während einer Kniegelenksoperation, mindestens ein erster Marker oder Referenzstern, ein aktiver, z.B. Infrarot-Strahlung oder sichtbares Licht emittierender Marker oder Referenzstern oder bevorzugt ein passiver, z.B. Infrarot-Strahlung oder sichtbares Licht reflektierender Marker oder Referenzstern, angebracht oder befestigt worden. Die Position oder Lage des mindestens einen ersten Markers oder Referenzsterns kann von einem Erfassungselement, wie z. B. einer IR-Kamera eines Navigationssystems, erfasst oder detektiert werden, so dass die Position des mindestens einen ersten Markers oder Referenzsterns im Raum bekannt ist oder ermittelt werden kann. Die Position mindestens eines ersten charakteristischen Punktes des ersten Objekts oder Knochens, vorzugsweise die Position von Landmarkenpunkten des ersten Objekts oder Knochens, wie charakteristischen Punkten des Femurs, zum Beispiel die Kondylen am distalen Ende des Femurs, charakteristischen Punkten der Tibia bzw. des Schienbeins, zum Beispiel die Eminenz am Tibia-Plateau, oder charakteristischen Punkten des Talus bzw. Sprungbeins, zum Beispiel die Mitte des Talus, kann mittels des mindestens einen ersten Markers oder Referenzsterns bei einem registrierten Objekt ermittelt werden. Auch kann die Position mindestens eines zweiten charakteristischen Punktes eines zweiten Objekts oder Knochens, wie des Femurs, der Tibia oder des Talus, welches mit dem ersten Objekt oder Knochen vorzugsweise über ein Gelenk, wie ein Kniegelenk, verbunden ist, z. B. mittels eines zweiten Markers oder Referenzsterns bestimmt werden, so dass die Lage oder Position des mindestens einen zweiten charakteristischen Punktes des zweiten Objekts oder Knochens relativ zu der Position des ersten charakteristischen Punktes des ersten Objekts ermittelt werden kann. Allgemein kann die Erfindung bei allen Objekten verwendet werden, welche ein sich innerhalb vorgegebener Grenzen bewegendes Lageverhältnis haben und z. B. durch ein Gelenk verbunden sind oder sein können.

Ändert sich die Position des ersten Markers oder Referenzsterns des ersten Objekts oder Knochens, zum Beispiel durch Versetzen oder Verdrehen des ersten Markers oder Referenzsterns und/oder die Position des zweiten Markers oder Referenzsterns des zweiten Objekts oder Knochens, zum Beispiel durch Versetzen oder Verdrehen des zweiten Markers oder Referenzsterns, so ändert sich die zum Beispiel von einer Erfassungseinheit erfasste oder von einer Recheneinheit ermittelte Position des ersten charakteristischen Punktes des ersten Objekts oder Knochens und/oder des zweiten charakteristischen Punktes des zweiten Objekts oder Knochens, so dass die mittels des Referenzsterns ermittelte Position des charakteristischen Punktes nicht mehr mit der tatsächlichen Position auf dem Objekt übereinstimmt. Aus der ermittelten Lage oder Position des mindestens einen ersten charakteristischen Punktes des ersten Objekts oder Knochens und aus der ermittelten oder bekannten Lage oder Position mindestens eines zweiten Punktes des zweiten Objekts oder Knochens kann ermittelt werden, ob sich die Position des mindestens einen ersten Markers oder Referenzsterns relativ zu dem ersten Objekt oder Knochen, an welchem der mindestens eine erste Marker oder Referenzstern angebracht ist, geändert hat. Zum Beispiel kann die relative Lage charakteristischer Punkte des ersten Objekts zu charakteristischen Punkten des zweiten Objekts bestimmt und mit anatomisch möglichen relativen Positionen verglichen werden, um zu bestimmen, ob die erfassten Positionen realistisch, also anatomisch möglich, sind, oder ob ein unrealistisches Lageverhältnis zwischen den Objekten vorliegt, wodurch ein Verdrehen oder Versetzen der Position mindestens eines Referenzsterns relativ zu einem Objekt festgestellt werden kann. Es kann auch bestimmt werden, ob sich die Lage oder Position des zweiten Markers oder Referenzsterns relativ zu dem zweiten Objekt oder Knochen, an welchem der zweite Marker oder Referenzstern befestigt ist, geändert hat.

Insbesondere kann aus der Lage der mindestens zwei charakteristischen Punkte im Raum die relative Lage oder Position der mindestens zwei charakteristischen Punkte zueinander ermittelt werden und aus der ermittelten relativen Lage der mindestens zwei charakteristischen Punkte zueinander bestimmt werden, ob sich die Position mindestens eines Markers oder Referenzsterns relativ zu dem ersten Objekt oder Knochen, an welchem der erste Marker oder Referenzstern angebracht oder befestigt ist, geändert hat, so dass z. B. ein Alarmsignal erzeugt werden kann.

Als erster charakteristischer Punkt kann zum Beispiel ein Punkt oder Endpunkt des ersten Objekts oder Knochens, wie Landmarkenpunkte eines Knochens, z.B. des Femurs, der Tibia oder des Talus, verwendet werden. Als zweiter charakteristischer Punkt kann ein Punkt oder Endpunkt des zweiten Objekts oder Knochens, wie Landmarkenpunkte des zweiten Knochens, z.B. des Oberschenkelknochens, des Schienbeins oder des Sprunggelenkknochens oder Sprungbeins verwendet werden. Vorzugsweise können auch Punkte, Endpunkte oder Fußpunkte einer das erste Objekt oder den ersten Knochen beschreibenden Achse oder einer das zweite Objekt oder den zweiten Knochen beschreibenden Achse, wie zum Beispiel mechanische Achsen des ersten oder zweiten Objekts oder Knochens dienen. Diese Achsen oder Geraden können zum Beispiel Verbindungen zwischen charakteristischen Punkten oder Landmarkenpunkten eines Objekts oder Knochens sein, wie zum Beispiel eine Achse oder Gerade durch eine Kondyle des Femurs und die Eminenz am Tibia-Plateau.

Insbesondere kann ein Abstand zwischen den mindestens zwei charakteristischen Punkten, zum Beispiel zwischen einem ersten Endpunkt bzw. Fußpunkt einer ersten das erste Objekt oder den ersten Knochen beschreibenden Achse, wie einer mechanische Achse des ersten Objekts oder Knochens, und einem zweiten Endpunkt bzw. Fußpunkt einer zweiten das zweite Objekt oder den zweiten Knochen beschreibenden Achse, wie einer mechanischen Achse des zweiten Objekts oder Knochens, ermittelt werden. Der ermittelte Abstand der mindestens zwei charakteristischen Punkte kann zum Beispiel mit zuvor ermittelten möglichen oder zulässigen Werten für Abstände der mindestens zwei charakteristischen Punkte oder mit vorgegebenen oder gespeicherten anatomisch möglichen Abständen verglichen werden und es kann bestimmt werden, ob der ermittelte Abstand der mindestens zwei charakteristischen Punkte den vorgegebenen oder gespeicherten oder zuvor ermittelten maximal möglichen Abstand oder Wert überschreitet. Insbesondere kann überprüft werden, ob der ermittelte Abstand der mindestens zwei charakteristischen Punkte größer als der vorgegebene Abstand oder Wert ist, wobei aus einer Überschreitung des vorgegebenen Abstandes oder Wertes geschlossen werden kann, dass die Position des mindestens einen ersten Markers oder Referenzsterns geändert wurde oder der mindestens eine erste Marker, zum Beispiel an eine andere Stelle des ersten Objekts oder Knochens, versetzt wurde oder dass der mindestens eine erste Marker oder Referenzstern um eine seiner Achsen, zum Beispiel um den Fußpunkt einer z. B. in der DE 201 03 416 U1 beschriebenen Vorrichtung zum Anbringen des mindestens einen ersten Markers oder Referenzsterns, verdreht oder verschoben wurde. Vorzugsweise kann zur Ermittlung oder Überprüfung des Abstandes der mindestens zwei charakteristischen Punkte einer der mindestens zwei charakteristischen Punkte als Mittelpunkt einer Kugel betrachtet werden, wobei der Radius der Kugel durch einen vorgegebenen oder gespeicherten oder zuvor ermittelten anatomisch möglichen Abstand oder Wert festgelegt wird, so dass aus einer Position oder Lage eines charakteristischen Punktes der mindestens zwei charakteristischen Punkte außerhalb der Kugel geschlossen werden kann, dass der Abstand zwischen den mindestens zwei charakteristischen Punkten den vorgegebenen maximal möglichen Abstand oder Wert überschreitet und sich die Position des mindestens einen ersten Markers oder Referenzsternes relativ zu dem ersten Objekt oder Knochen berücksichtigt hat. Der vorgegebene Abstand oder Wert kann zum Beispiel 0,1 mm bis zu 5 cm betragen, je nachdem welche Objekte oder Knochen berücksichtigt werden, oder wie die Objekte oder Knochen verbunden sind. Ist die Verbindung zum Beispiel ein Gelenk, wie ein Kniegelenk, muss zur Bestimmung des anatomisch maximal möglichen Abstandes zweier Endpunkte der an das Gelenk angrenzenden Knochen eine Verschiebung der Position der mindestens zwei charakteristischen Punkte zum Beispiel aufgrund der bestehenden Flexibilität von Bändern oder Sehnen, die dazu führt, dass sich die Knochen gegeneinander verschieben oder verdrehen können, berücksichtigt werden.

Bevorzugt kann auch die Position eines weiteren charakteristischen Punktes des ersten Objekts oder Knochens, wie eines Landmarkenpunktes des ersten Knochens, und eines weiteren charakteristischen Punktes des zweiten Objekts oder Knochens, wie eines Landmarkenpunktes des zweiten Knochens, erfasst oder bestimmt werden und zum Beispiel von einer Recheneinheit weiter verarbeitet werden, um z. B. zu untersuchen, ob die durch die Lage der Punkte definierte relative Lage der den Objekten zugeordneten Achsen anatomisch möglich ist. Es kann in der Recheneinheit aus den mindestens zwei charakteristischen Punkten des ersten Objekts oder Knochens eine Achse des ersten Objekts, wie eine mechanische Achse des ersten Objekts oder Knochens oder eine Achse oder Gerade durch Landmarkenpunkte des ersten Knochens ermittelt werden und aus den mindestens zwei charakteristischen Punkten des zweiten Objekts oder Knochens eine Achse des zweiten Objekts, zum Beispiel eine mechanische Achse des zweiten Objekts oder eine Achse oder Gerade durch Landmarkenpunkte des zweiten Knochens, bestimmt werden, wobei die Lage oder Position der Achse des ersten Objekts relativ zu der Lage der Achse des zweiten Objekts untersucht oder ermittelt wird. Aus der relativen Lage der beiden Achsen zueinander kann darauf geschlossen werden, ob sich die Position des mindestens einen ersten Markers oder Referenzsterns relativ zu dem ersten Objekt oder Knochen, an welchem der erste Marker oder Referenzstern angebracht ist, geändert hat oder verändert wurde.

Vorzugsweise kann zum Beispiel die Richtung der beiden Achsen mit vorgegebenen Richtungen oder gespeicherten Richtungen zweier Achsen im Raum oder mit zuvor ermittelten und gespeicherten Richtungen oder anatomisch möglichen relativen Lagen der Achsen des ersten und zweiten Objekts verglichen werden und aus dem Vergleich darauf geschlossen werden, ob sich zum Beispiel die Lagen außerhalb eines anatomisch möglichen Bereichs befinden. Daraus kann gefolgert werden, dass, wenn die Lage der beiden Objekte zueinander, zum Beispiel aufgrund einer bekannten relativen Lagerung der beiden Objekte oder Knochen zueinander durch ein beide Objekte verbindendes Gelenk, nicht geändert wurde, eine Änderung der Position des mindestens einen ersten Markers oder Referenzsterns relativ zu dem ersten Objekt oder Knochen stattgefunden hat.

Bevorzugt kann auch ein durch die beiden Objektachsen oder Knochenachsen definierter Winkel ermittelt werden, zum Beispiel der von den beiden Objektachsen oder Knochenachsen eingeschlossene Winkel am Schnittpunkt der Objekt- oder Knochenachsen, und aus dem ermittelten Winkel, vorzugsweise durch einen Vergleich des Winkels mit vorgegebenen oder bekannten oder zuvor ermittelten und gespeicherten Winkelwerten, bestimmt werden, ob sich die Position des mindestens einen ersten Markers oder Referenzsterns relativ zu dem ersten Objekt oder Knochen, an dem der erste Marker oder Referenzstern angeordnet ist, geändert hat. Vorzugsweise wird dabei zum Beispiel in der Recheneinheit überprüft, ob der ermittelte Winkel bzw. Winkelwert größer als ein vorgegebener oder zuvor ermittelter anatomisch maximal möglicher Winkelwert ist. Aus der Überschreitung des vorgegebenen oder zuvor ermittelten Winkelwertes, der zum Beispiel in einer Datenbank oder einem Speicher als Absolutwert oder patientenspezifisch gespeichert sein kann oder werden kann, kann zum Beispiel in der Recheneinheit erkannt werden, dass eine Positionsänderung des mindestens einen ersten Markers stattgefunden hat, insbesondere dass der mindestens eine erste Marker versetzt oder um eine Achse gedreht wurde.

Wird eine Positionsänderung des mindestens einen ersten Markers oder Referenzsterns mit einem der bislang beschriebenen Verfahren erkannt, kann ein Warnsignal ausgegeben werden, welches angibt, dass eine Positionsänderung des mindestens einen ersten Markers oder Referenzsterns relativ zu dem ersten Objekt stattgefunden hat. Dieses Warnsignal kann zum Beispiel ein akustisches oder ein visuelles bzw. optisches Signal sein. Das visuelle Signal könnte zum Beispiel die Position des mindestens einen ersten Markers oder Referenzsterns, insbesondere relativ zu dem ersten Objekt, an welchem der erste Marker oder Referenzstern befestigt ist, z.B. auf einem Bildschirm oder Computer, anzeigen, so dass ein Chirurg erkennen kann, dass eine ungewollte Positionsänderung des mindestens einen Markers oder Referenzsterns vorliegt. Insbesondere kann zum Beispiel auf dem Bildschirm mittels eines Zahlenwertes oder einer grafischen Darstellung angezeigt werden, um wie viel sich der mindestens eine erste Marker oder Referenzstern verschoben oder versetzt oder verdreht hat, so dass der Operateur diese Positionsänderung rückgängig machen kann.

Vorteilhaft kann z. B. auf einem Objekt oder Knochen eine Markierung angebracht sein, welche eine einfache Neupositionierung des Referenzsterns ermöglicht, ohne eine Neuregistrierung durchführen zu müssen, z. B. indem eine an einem Referenzsternhalter angebrachte Markierung in Deckung mit der Objektmarkierung gebracht wird.

Vorzugsweise wird in vorgegebenen Abständen, die insbesondere auch regelmäßig oder gleichmäßig sein können, die Position des mindestens einen ersten Markers oder Referenzsterns überprüft. Dabei kann die Wiederholung zum Beispiel jede Sekunde durchgeführt werden oder in einem Intervall, welches zwischen 1 ms und 10s liegen kann, ausgeführt werden.

Die Erfindung bezieht sich des Weiteren auf ein Computerprogramm, welches, wenn es in einen Computer geladen wird oder auf einem Computer läuft, ein wie oben beschriebenes Verfahren durchführt. Weiterhin bezieht sich die Erfindung auf ein Programmspeichermedium oder ein Computerprogrammprodukt mit einem solchen Programm.

Eine erfindungsgemäße Vorrichtung zur Erkennung einer Positionsänderung mindestens eines Markers oder Referenzsterns relativ zu einem Objekt oder Knochen, an welchem der Marker oder Referenzstern befestigt oder angebracht ist, umfasst ein erstes Erfassungselement, insbesondere eine Infrarot-Kamera, welche die Lage oder Position mindestens eines charakteristischen Punktes eines ersten Objekts oder Knochens, zum Beispiel eines Landmarkenpunkts des ersten Knochens, erfassen kann, da die Lage des mindestens einen charakteristischen Punktes relativ zu einem an dem ersten Objekt angebrachten Marker oder Referenzstern nach der Registrierung des Objekts bekannt ist oder bezüglich des mindestens einen an dem ersten Objekt oder Knochen angebrachten Markers oder Referenzsterns definiert werden kann. Somit können Veränderungen der Lage oder Position des ersten Objekts oder Knochens, wie Bewegungen des ersten Objekts oder Knochens, von dem ersten Erfassungselement erfasst werden, woraus auf die dreidimensionale Position oder Lage des mindestens einen charakteristischen Punktes im Raum geschlossen werden kann.

Die erfindungsgemäße Vorrichtung umfasst weiter ein zweites Erfassungselement, welches z. B. das erste Erfassungselement sein kann oder ein Positionierungselement zur z. B. festen Lagerung des zweiten Objektes ist. Mit Hilfe des zweiten Erfassungselementes kann die Lage oder Position mindestens eines charakteristischen Punktes eines zweiten Objekts erfasst oder ermittelt werden. Vorzugsweise kann auf dem zweiten Objekt oder Knochen mindestens ein Marker oder Referenzstern angeordnet sein, der von dem Erfassungselement erfasst werden kann.

Die erfindungsgemäße Vorrichtung umfasst ferner eine Recheneinheit, welche mit dem ersten Erfassungselement verbunden ist und z.B. die relative Lage der mindestens zwei charakteristischen Punkte aus den von dem Erfassungselement erfassten räumlichen Positionen der charakteristischen Punkte ermittelt. Aus der ermittelten relativen Lage der mindestens zwei charakteristischen Punkte kann die Recheneinheit zum Beispiel durch Vergleich der ermittelten Lage mit in einer Datenbank oder einem Speicher gespeicherten Lagen oder Positionen der mindestens zwei charakteristischen Punkte bestimmen, ob sich einer der Marker oder Referenzsterne relativ zu dem ersten Objekt bewegt hat.

Die Vorrichtung zur Erkennung einer Positionsänderung mindestens eines Markers oder Referenzsterns relativ zu dem Objekt, an welchem der Marker oder Referenzstern befestigt ist, kann auch eine Datenausgabevorrichtung, insbesondere eine Warnvorrichtung in Gestalt eines Bildschirms oder eines Lautsprechers, umfassen, welche mit der Recheneinheit verbunden sein können und ein Warnsignal ausgibt, wenn eine ungewollte Positionsänderung des mindestens einen Markers oder Referenzsterns erkannt wird.

Die Erfindung wird nachfolgend anhand bevorzugter Ausführungsbeispiele beschrieben werden. Es zeigen:
- Figur 1: eine Vorrichtung der vorliegenden Erfindung in einem ersten Zustand;
- Figur 2: die Vorrichtung aus Figur 1 in einem zweiten Zustand.

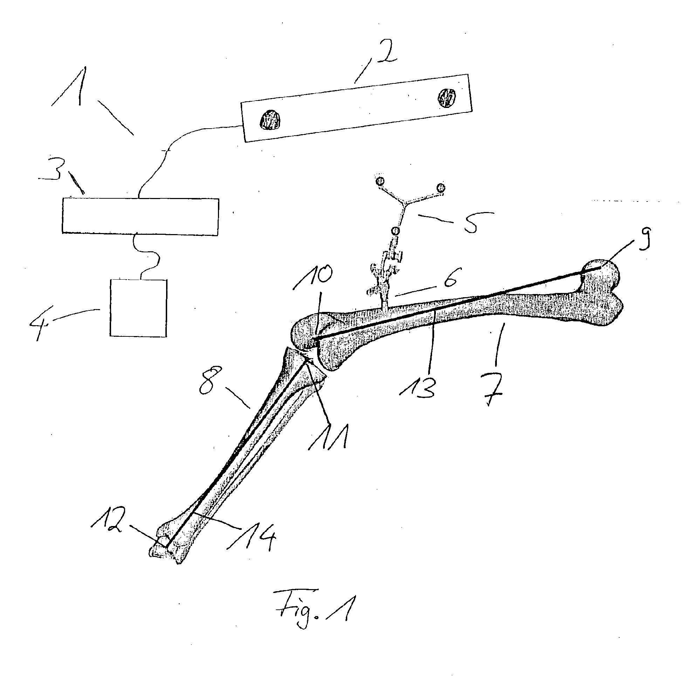

Figur 1 zeigt eine Vorrichtung 1 der vorliegenden Erfindung, wobei das erste Objekt 7 ein Oberschenkelknochen bzw. Femur 7 und das zweite Objekt 8 ein Schienbein bzw. eine Tibia 8 ist, mit einer Infrarot-Kamera 2 als Erfassungselement, einer Recheneinheit 3, die mit der Infrarot-Kamera 2 verbunden ist, und einem Bildschirm 4 als Datenerfassungsvorrichtung. Auf dem Oberschenkelknochen bzw. Femur 7 ist ein Referenzstern 5 angebracht. Vorzugsweise ist der Referenzstern 5 mit Hilfe der Befestigungsvorrichtung 6 gemäß der DE 20 103 416.6 U1, deren Lehre bezüglich der Ausgestaltung einer solchen Vorrichtung 6 in diese Anmeldung aufgenommen wird, an dem Femur 7 befestigt. Wird zum Beispiel der Referenzstern 5 oder die Befestigungsvorrichtung 6 zum Befestigen des Referenzsterns 5 während einer Operation berührt, kann sich die Position des Referenzsterns 5 zum Beispiel durch Verdrehen der Vorrichtung 6 relativ zum Femur 7 verändern. In dem in Figur 1 gezeigten ersten Zustand sind zwei charakteristische Punkte 9, 10 oder Landmarkenpunkte des Oberschenkelknochens 7 an den Enden des Oberschenkelknochens 7 bezüglich des Referenzsterns 5 registriert, wobei in der Recheneinheit 3 virtuell, wie in Figur 1 zu sehen, eine Achse 13 oder Gerade zwischen den zwei charakteristischen Punkten 9, 10 gezogen wird. Eine zweite Achse 14 wird von der Recheneinheit 3 durch zwei charakteristische Punkte 11, 12 oder Landmarken des Schienbeins 8 gezogen, wobei die charakteristischen Punkte 11, 12 des Schienbeins 8 bezüglich eines weiteren Referenzsterns (nicht gezeigt) oder durch ortsfeste Positionierung der Tibia 8 registriert sind.

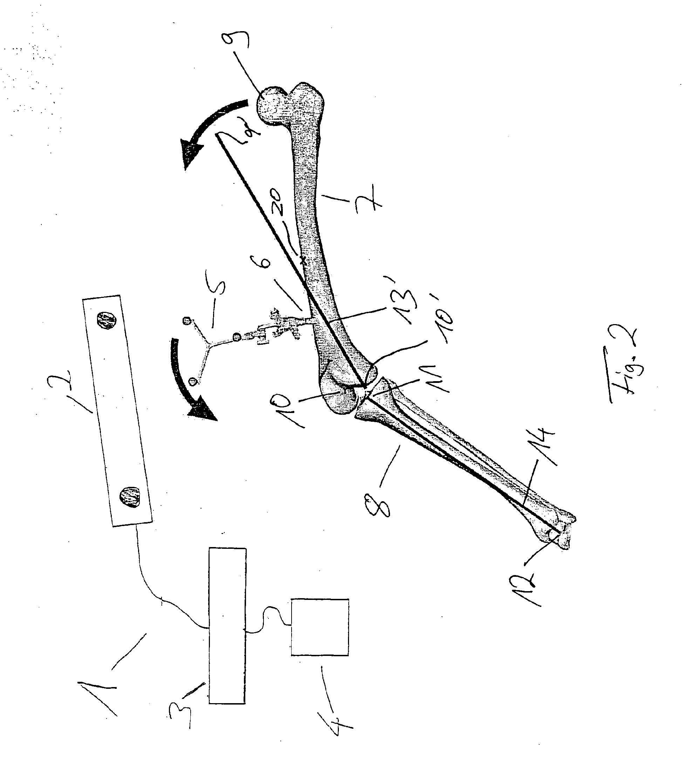

Wird der Referenzstern 5 zum Beispiel durch eine Berührung des Chirurgen unbeabsichtigt gedreht, wie in Figur 2 dargestellt, ermittelt die Recheneinheit 3 durch die von der Infrarot-Kamera 2 erfassten Positionen der Marker des Referenzsternes 5 eine falsche Position für die charakteristischen Punkte 9' und 10', welche von der tatsächlichen Position der charakteristischen Punkte 9, 10 des Femurs 7 abweicht, wodurch sich die ermittelte Achse 13' des Femurs 7, wie in Figur 2 gezeigt, verschiebt. Die Recheneinheit 3 erkennt durch einen Vergleich mit vorher abgespeicherten anatomisch möglichen Relativpositionen der mechanischen Femur- und Tibia-Achsen, dass sich die relative Lage der den Oberschenkelknochen 7 beschreibenden Achse 13' und der das Schienbein 8 beschreibenden Achse 14 außerhalb eines anatomisch möglichen Bereichs bewegt hat und schließt daraus, dass sich die Lage des Referenzsterns 5 bezüglich des Oberschenkelknochens 7 geändert hat oder der Referenzstern 5 relativ zu dem Oberschenkelknochen 7 verdreht wurde. Darauf gibt ein mit der Recheneinheit 3 verbundener Bildschirm 4 zum Beispiel ein Warnsignal für den Arzt aus, das anzeigt, dass sich der Referenzstern 5 verdreht hat oder zeigt zum Beispiel mittels einer grafischen Darstellung auf dem Bildschirm 4 an, um wie viel sich der Referenzstern 5 verdreht hat oder wieder zurückgedreht werden muss. Auch kann der Referenzstern 5 mittels einer Markierung 20 auf dem Femur 7 in die Ausgangsposition, die der Referenzstern 5 vor der Veränderung der Position oder der Verdrehung hatte, zurückgedreht werden.

## Patentansprüche

1. Verfahren zur Erkennung einer Positionsänderung mindestens eines an einem ersten Objekt (7) angebrachten ersten Markers oder Referenzsternes (5) relativ zu dem ersten Objekt (7), wobei die Lage mindestens eines ersten charakteristischen Punktes (10) des ersten Objekts (7) mittels des daran befestigten ersten Markers oder Referenzsterns (5) ermittelt wird, die Lage mindestens eines zweiten charakteristischen Punktes (11) eines mit dem ersten Objekt (7) verbundenen zweiten Objekts (8) ermittelt wird und aus der ermittelten Lage der mindestens zwei charakteristischen Punkte (10, 11) bestimmt wird, ob sich die Position des mindestens einen ersten Markers oder Referenzsterns (5) relativ zu dem ersten Objekt (7), an welchem der erste Marker oder Referenzstern (5) befestigt ist, geändert hat.

2. Verfahren nach dem vorhergehenden Anspruch, wobei als erster charakteristischer Punkt (10) ein Endpunkt des ersten Objekts (7) oder einer das erste Objekt (7) beschreibenden Achse (13) verwendet wird und als zweiter charakteristischer Punkt (11) ein Endpunkt des zweiten Objekts (8) oder einer das zweite Objekt (8) beschreibenden Achse (14) verwendet wird.

3. Verfahren nach einem der vorhergehenden Ansprüche, wobei die relative Lage der mindestens zwei charakteristischen Punkte (10, 11) zueinander ermittelt wird und aus der ermittelten relativen Lage der mindestens zwei charakteristischen Punkte (10, 11) zueinander bestimmt wird, ob sich die Position des mindestens einen ersten Markers oder Referenzsterns (5) relativ zu dem ersten Objekt (7), an welchem der erste Marker oder Referenzstern (5) befestigt ist, geändert hat.

4. Verfahren nach einem der vorhergehenden Ansprüche, wobei ein Abstand zwischen den mindestens zwei charakteristischen Punkten (10, 11) ermittelt wird und bestimmt wird, ob der ermittelte Abstand der mindestens zwei charakteristischen Punkte (10, 11) einen vorgegebenen Abstand überschreitet und aus der Überschreitung des vorgegebenen Abstandes erkannt wird, dass sich die Position des mindestens einen ersten Markers oder Referenzsternes (5) relativ zu dem ersten Objekt (7), an welchem der erste Marker oder Referenzstern (5) befestigt ist, geändert hat.

5. Verfahren nach einem der vorhergehenden Ansprüche, wobei ein weiterer charakteristischer Punkt (9) des ersten Objekts (7) bestimmt wird, wobei der erste charakteristische Punkt (10) und der weitere charakteristische Punkt (9) des ersten Objekts (7) eine Achse (13) des ersten Objekts (7) definieren, ein weiterer charakteristischer Punkt (12) des zweiten Objekts (8) bestimmt wird, wobei der zweite charakteristische Punkt (11) und der weitere charakteristische Punkt (12) des zweiten Objekts (8) eine Achse (14) des zweiten Objekts (8) definieren und aus der Lage der Achse (13) des ersten Objekts (7) relativ zu der Lage der Achse (14) des zweiten Objekts (8) ermittelt wird, ob sich die Position mindestens eines ersten Markers oder Referenzsternes (5) relativ zu dem ersten Objekt (7), an welchem der erste Marker oder Referenzstern (5) befestigt ist, geändert hat.

6. Verfahren nach dem vorhergehenden Anspruch, wobei ein durch die beiden Objektachsen (13, 14) definierter Winkel ermittelt wird und aus dem ermittelten Winkel, insbesondere aus der Überschreitung eines vorgegebenen Winkelwertes, erkannt wird, ob sich die Position des mindestens einen ersten Markers oder Referenzsternes (5) relativ zu dem ersten Objekt (7), an welchem der erste Marker oder Referenzstern (5) befestigt ist, geändert hat.

7. Verfahren nach dem vorhergehenden Anspruch, wobei der Marker oder Referenzstern (5) durch eine an dem Objekt (7) angebrachte Markierung (20) in die Ausgangsposition zurückgebracht oder -gedreht wird.

8. Verfahren nach einem der vorhergehenden Ansprüche, wobei bei Erkennung der Positionsänderung des mindestens einen ersten Markers oder Referenzsternes (5) relativ zu dem ersten Objekt (7), an welchem der erste Marker oder Referenzstern (5) befestigt ist, ein Signal, insbesondere ein Warnsignal ausgegeben wird.

9. Verfahren nach einem der vorhergehenden Ansprüche, wobei das Verfahren in vorgegebenen Abständen, insbesondere in einem Intervall zwischen 1 Millisekunde und 10 Sekunden, wiederholt wird.

10. Computerprogramm, welches, wenn es auf einem Computer läuft oder in einen Computer geladen ist, das Verfahren nach einem der vorhergehenden Ansprüche durchführt.

11. Programmspeichermedium oder Computerprogrammprodukt mit dem Computerprogramm nach dem vorhergehenden Anspruch.

12. Vorrichtung (1) zur Erkennung einer Positionsänderung mindestens eines Markers oder Referenzsterns (5) mit:
- einem ersten Erfassungselement (2) zur Erfassung der Lage mindestens eines charakteristischen Punktes (10) eines ersten Objekts (7) mittels eines an dem ersten Objekt (7) angebrachten Markers oder Referenzsterns (5);
- einem zweiten Erfassungselement zur Bestimmung der Lage mindestens eines charakteristischen Punktes (11) eines zweiten Objekts (8); und
- einer Recheneinheit (3), welche mit dem ersten Erfassungselement (2) verbunden ist, zur Ermittlung der relativen Lage der mindestens zwei charakteristischen Punkte (10, 11), wobei anhand der relativen Lage der mindestens zwei charakteristischen Punkte (10, 11) ermittelt werden kann, ob sich einer der Marker oder Referenzsterne (5) relativ zu dem ersten Objekt (7) bewegt hat.

13. Vorrichtung nach dem vorhergehenden Anspruch, wobei an dem zweiten Objekt (8) mindestens ein Marker oder Referenzstern (5) angebracht ist, woraus das relative Lageverhältnis des zweiten Objektes (8) relativ zu dem ersten Objekt (7) bekannt ist.

14. Vorrichtung nach einem der zwei vorhergehenden Ansprüche, wobei das erste Erfassungselement (2) eine Infrarot-Kamera (2) ist.

15. Vorrichtung nach einem der drei vorhergehenden Ansprüche, wobei das zweite Erfassungselement eine Infrarot-Kamera (2) ist, welche mit der Recheneinheit (3) verbunden ist, oder ein Positionierungselement ist.

16. Vorrichtung nach einem der vier vorhergehenden Ansprüche mit einer Datenausgabevorrichtung (4), insbesondere einer Warnvorrichtung, die mit der Recheneinheit (3) verbunden ist, zur Ausgabe eines Signals, insbesondere eines Warnsignals, wenn die Positionsänderung des mindestens einen Markers oder Referenzsternes (5) relativ zu dem Objekt (7) erkannt wird.

17. System zur Erkennung einer Positionsänderung mindestens eines Markers oder Referenzsterns (5) relativ zu einem damit verbundenen Objekt (7) mit einer Vorrichtung nach einem der fünf vorhergehenden Ansprüche und mindestens einem Marker oder Referenzstern (5).
